(19) Europäisches Patentamt
European Patent Office
Office européen des brevets

(11) Veröffentlichungsnummer : **0 356 869 B1**

(12) **EUROPÄISCHE PATENTSCHRIFT**

(45) Veröffentlichungstag der Patentschrift :
**19.11.92 Patentblatt 92/47**

(51) Int. Cl.$^5$ : **C07C 403/08**

(21) Anmeldenummer : **89115416.3**

(22) Anmeldetag : **21.08.89**

(54) **Verfahren zur Herstellung eines cyclischen, ungesättigten Ketons.**

(30) Priorität : **30.08.88 CH 3216/88**
**22.06.89 CH 2324/89**

(43) Veröffentlichungstag der Anmeldung :
**07.03.90 Patentblatt 90/10**

(45) Bekanntmachung des Hinweises auf die Patenterteilung :
**19.11.92 Patentblatt 92/47**

(84) Benannte Vertragsstaaten :
**CH DE ES FR GB IT LI NL**

(56) Entgegenhaltungen :
**DE-A- 3 516 931**
**US-A- 4 072 719**
**CHEMICAL ABSTRACTS, Band 96, Nr. 25, 21. Juni 1982, Seite 693, Spalte 2, Zusammenfassung Nr. 217339j, Columbus, Ohio, USA;& JP-A-57/026637**

(73) Patentinhaber : **L. GIVAUDAN & CIE Société Anonyme**
**CH-1214 Vernier-Genève (CH)**

(72) Erfinder : **Helmlinger, Daniel**
**Ticheirütistrasse 18**
**CH-8044 Gockhausen (CH)**
Erfinder : **Kienzle, Frank**
**Tannwaldweg 9**
**CH-4113 Flüh (CH)**
Erfinder : **Widmer, Erich**
**Mittelweg 47**
**CH-4142 Münchenstein (CH)**

(74) Vertreter : **Urech, Peter, Dr. et al**
**Grenzacherstrasse 124 Postfach 3255**
**CH-4002 Basel (CH)**

**EP 0 356 869 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von 4-(2-Butenyliden)-3,5,5-trimethyl-2-cyclohexen-1-on (I) (Megastigmatrienon).

Genauer gesagt, betrifft die Erfindung ein Verfahren zur Herstellung von Gemischen der 4 geometrischen Isomeren von Megastigmatrienon,

nämlich der Verbindungen

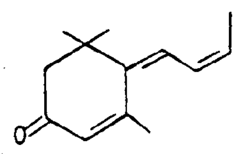
Ia

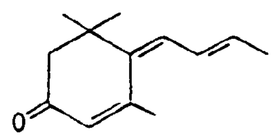
Ib

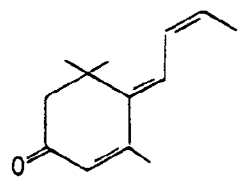
Ic

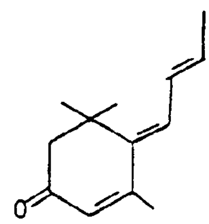
Id

Das Verfahren ist dadurch gekennzeichnet, dass man eine Verbindung der Formel

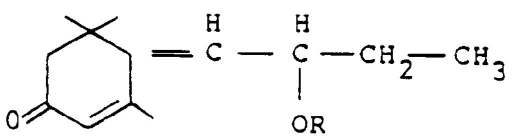
II

worin R nieder-Alkanoyl, d.h. $C_{1-6}$-Alkanoyl, oder gegebenenfalls substituiertes (z.B. durch nieder-Alkyl, wie Methyl) Benzoyl, oder nieder-Alkyl-oxycarbonyl

$$(R'-O-\overset{O}{\overset{\|}{C}}-)$$

oder Aryl-oxycarbonyl

$$(R''-O-\overset{O}{\overset{\|}{C}}-)$$

darstellt, insbesondere ein Gemisch der beiden Isomeren der Formeln

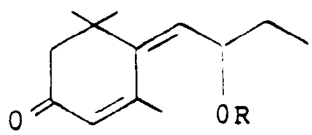

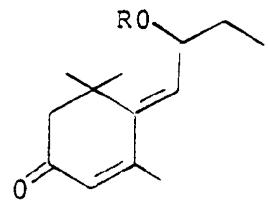

IIa (Z) IIb (E)

pyrolysiert.

Beispiele von Resten R sind Acetyl, Propionyl, Butyryl, Caproyl, Benzoyl, Toluyl, etc. Bevorzugt ist Acetyl. R′ ist $C_{1-6}$-Alkyl, z.B. Methyl, Aethyl, etc. Bevorzugte Reste R″ sind Phenyl und Tolyl.

Die Verbindung II bzw.deren Isomere werden zweckmässigerweise durch Veresterung von 4-(2-Hydroxybutyliden)-3,5,5-trimethyl-2-cyclohexen-1-on (III)

bzw. des Isomerengemisches

und

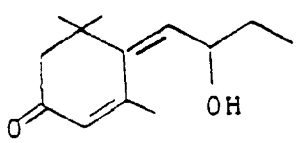

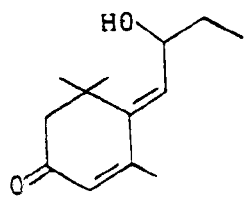

IIIa (Z) IIIb (E)

hergestellt.

Die Verbindung III bzw. deren Isomerengemische sind durch Umsetzung des 2,6,6-Trimethyl-4-oxo-2-cyclohexen-1-yliden)acetaldehyds

bzw. den Isomeren

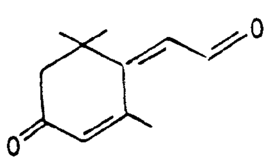

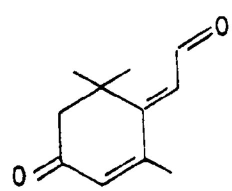

IVa (Z) IVb (E)

mit einem organometallischen Reagens zugänglich.

Es sind bereits eine Reihe Synthesen zur Herstellung von Megastigmatrienon bekannt geworden, siehe z.B.

[1] US-PS 3211 157

[2] US-PS 3,217,718

[3] E. Demole, P. Enggist, Helv. Chim. Acta 57, 2087-2089, (1974)

[4] B.M. Trost, J.L. Stanton, JACS 97, 4018-4025, (1975)

[5] S. Torrii, J. Inokuchi, H. Ogawa, Bull. Chem. Soc. Japan 52, 1233-1234, (1979)

[6] O. Takazawa, H. Tamura, K. Kogami, K. Hayashi, Bull. Chem. Soc. Japan 55, 1907, (1982)

[7] Deutsche Offenlegungsschrift 35 16 931

Alle diese Synthesen sind mit Nachteilen behaftet, wie z.B.

Benutzung problematischer Reagenzien (2), (7),
uneinheitliche Reaktionsprodukte (3),
teure Reagenzien (4), (5), (6),
schwer zugängliche Ausgangsmaterialien (1).

Dem neuen Verfahren haftet keiner dieser Nachteile an. Ein weiterer Vorteil der gegenständlichen Synthese besteht darin, dass die Isomerenverteilung in I nahe an die Isomerenverteilung herankommt, wie sie in der Natur vorkommt, siehe z.B. T. Fujimori, R. Kasuga, H. Kaneko, M. Noguchi, Central Research Institute, The Japan Tobacco and Salt Public Corporation, Yokohama, Japan; Presentation at the 7th International Congress of Essential Oils, Kyoto, Japan, 1977, siehe Fig. 4 "Gas chromatogramm of the neutral volatile fraction of leaves before curing and aging"; siehe ferner Tobacco Report, Oct. 1983, 42.

Die Pyrolyse der Verbindung II wird zweckmässigerweise bei Temperaturen von ca. 400 - 600 °C, insbesondere bei ca. 450 - 550°C durchgeführt. Man arbeitet zweckmässigerweise unter Inertgasatmosphäre, z.B. unter Stickstoff oder in Edelgasatmosphäre.

Die Reinigung von I erfolgt zweckmässigerweise durch Destillation.

Die Veresterung von 4-(2-Hydroxybutyliden)-3,5,5-trimethyl -2-cyclohexen-1-on erfolgt zweckmässigerweise unter Verwendung üblicher Acylierungsmittel, also z.B. Acylhalogeniden, insbesondere des Chlorids, oder Säureanhydriden. Bevorzugt ist die Arbeitsweise unter Verwendung der Säureanhydride. Man arbeitet zweckmässigerweise in Anwesenheit einer Base, z.B. eines organischen Amins wie Pyridin oder Dimethylanilin, und gegebenenfalls unter Verwendung eines inerten Lösungsmittels, z.B. eines Kohlenwasserstoffes oder eines Aethers, z.B. Hexan, Cyclohexan, Toluol, Diäthyläther, etc. Ein geeigneter Temperaturbereich ist derjenige von etwa 20°C - 80°C;
siehe auch Organikum, Org. chem. Grundpraktikum VEB Deutscher Verlag der Wissenschaften, Berlin (1986), Seiten 402 ff.

Die Herstellung der Hydroxyverbindung (III) erfolgt erfindungsgemäss durch Reaktion des Ketoaldehyds IV mit einem organometallischen Reagens, welches die Seitenkette an der Aldehydfunktion um 2 Kohlenstoffatome verlängert. Ein solches zweckmässiges Reagens ist beispielsweise zugänglich durch Umsetzung von

$C_2H_5$ MgX mit einem Reagens der Formel $MeX_2$, von
$C_2H_5$ MgX und $(C_2H_5)_2$ Me, oder
mit $(C_2H_5)_2Me$
worin Me = Zink oder Cadmium darstellt und X Brom, Chlor oder Jod ist.

Bevorzugt ist $C_2H_5MgCl$ / $ZnCl_2$.

Die erfindungsgemässe Reaktion wird in an sich bekannter Weise durchgeführt, also z.B. durch Zugabe des Halogenids $C_2H_5MgX$ in Aether, zweckmässigerweise bei Raumtemperatur, zum Halogenid $MeX_2$. Dazu gibt man zweckmässigerweise eine ätherische Lösung von IV. Die Aufarbeitung geschieht durch Extraktion von III mit einem organischen Lösungsmittel; siehe auch Organic Reactions VIII, 31; J. Wiley, New York-London (1954).

Der Ketoaldehyd IV ist bekannt, siehe z.B.

H. Mayer, M. Montavon, R. Ruegg, O. Isler, Helv. chim. Acta 50, Fasc. 6 (1967), Seite 1606, oder US PS 2827 481

Ein für die vorliegende Synthese besonders geeignetes Isomerengemisch von IV besteht zu ca. 25 bis 35% aus dem E-Isomeren und zu ca. 75 bis 65% aus dem Z-Isomeren.

Ein solches Gemisch ist beispielsweise aus 3,5,5-Trimethyl-4-hydroxy-4-aethinyl-cyclohex-2-en-1-on durch dessen Umlagerung mittels Katalysatoren auf der Basis von Silylvanadaten in an sich bekannter Weise leicht zugänglich. Bevorzugt ist tris(Triphenylsilyl)vanadat; siehe auch L. Cerveny und Vlastimil Ruzicka, Parfümerie und Kosmetik, 69 (1), 9, (1988).

Die Isomerenverteilung von IV wird in den oben beschriebenen Folgestufen im wesentlichen beibehalten.

Die Verbindungen II und III als auch deren Gemische sind neu und bilden einen weiteren Gegenstand der Erfindung.

## Beispiel

a) Zu 900 ml Ammoniak werden bei -35°C 15 g (2,2 M) Lithium in kleinen Stücken innerhalb einer Stunde zugegeben. Es wird Acetylen durchgeleitet, bis die blaue Farbe verschwindet und eine graue Suspension entsteht. Dann werden 150 g (1 M) Ketoisophoron zugetropft. Innert 12 Stunden wird das Ammoniak abgedampft. Es verbleibt ein orangefarbiger Rückstand. Anschliessend wird 1 Liter Eiswasser zugegeben und unter Rühren wird mit Aether versetzt, und zunächst mit 2N HC1 und hierauf mit konz. HCl sauer gestellt. Die Phasen werden getrennt und die wässrige Phase wird mit Aether viermal extrahiert. Es wird zweimal mit Wasser und mit gesättigter Kochsalzlösung gewaschen, getrocknet, filtriert und eingeengt. Man erhält auf diese Weise 166 g rohes 3,5,5-Trimethyl-4-hydroxy-4-aethinyl-cyclohex-2-en-l-on. Dieses Rohprodukt wird in 500 ml Aether fast vollständig gelöst und dann auf -25° abgekühlt. Man erhält auf diese Weise 86.2 g (48%) kristallines 3,5,5-Trimethyl-4-hydroxy-4-aethinyl-cyclohex-2-en-l-on. Die Mutterlauge wird in 140 ml Isopropylaether gelöst und auf 0° abgekühlt. Man erhält auf diese Weise nochmals 25 g (14%) Kristalle der Zielverbindung.

b) 22 g Triphenylsilanol, 80 g (0,45 M) 3,5,5-Trimethyl-4-hydroxy-4-aethinyl-cyclohex-2-en-l-on und 1,6 g Stearinsäure werden in 800 ml Xylol gelöst, mit 1,92 ml Vanadiumisopropylat versetzt und unter Rühren auf Rückflusstemperatur erhitzt. Nach 3 1/2 Stunden wird abgekühlt und das Reaktionsgemisch mit 5% Natriumbicarbonat gewaschen, wobei eine Emulsion entsteht. Die organische Phase wird zunächst abgetrennt, auch die Emulsion wird abgetrennt und mit Aether extrahiert. Die organischen Phasen werden über Magnesiumsulfat getrocknet und eingeengt. Der Rückstand (117,9 g) wird destilliert (Oelbad 130-170°; 0,133 Pa (0,001 mm Hg)). Man erhält auf diese Weise 51,8 g (64,8%) (2,6,6-Trimethyl-4-oxo-2-cyclohexen-1-yliden)acetaldehyd (Z:E = ca. 3:1).

c) 122,6 g (0,786 M) Aethyliodid in 100 ml Aether werden innert 40 Minuten zu 19,1 g (0,786 M) Magnesium in Aether zugetropft. Man lässt 12 Stunden bei Raumtemperatur unter Rühren reagieren. Das gebildete Reagens wird zu 107,14 g (0,786 M) Zinkchlorid in 400 ml Aether innert 40 Minuten zugetropft (leicht exotherme Reaktion). Man lässt unter Rühren 1 1/2 Stunden bei Rückflusstemperatur reagieren. Dann werden 100 g (0,562 M) (2,6,6-Trimethyl-4-oxo-2-cyclohexen-1-yliden)acetaldehyd gelöst in 400 ml Aether innert 30 Minuten bei Raumtemperatur unter Rühren zugegeben. Nach der Hälfte der Zugabe bildet sich ein dickflüssiger Brei, welcher sich aber wieder löst. Anschliessend wird 4 Stunden bei Rückfluss und 48 Stunden bei Raumtemperatur gerührt. Das Reaktionsgemisch wird auf Eis gegossen und mit Aether extrahiert. Die organische Lösung wird dreimal mit je 300 ml Wasser, dann mit 300 ml gesättigter Kochsalzlösung neutral gewaschen. Die organische Phase wird über Magnesiumsulfat getrocknet und eingedampft. Man erhält 104 g eines Gemisches von (Z)-4-(2-Hydroxybutyliden)-3,5,5-trimethyl-2-cyclohexen-1-on und (E)-4-(2-Hydroxybutyliden)-3,5,5-trimethyl-2-cyclo-hexen-1-on, das sofort weiter umgesetzt wird.

d) Zu 104 g (0,5 M) des obigen Gemisch in 500 ml Pyridin werden bei Raumtemperatur 54,9 g (0,54 M) Acetanhydrid zugegeben. Nach 18 Stunden Rühren bei Raumtemperatur wird das Pyridin abdestilliert. Der Rückstand wird in Aether gelöst und zweimal mit 2N HCl, mit gesättigter Natriumbicarbonatlösung, mit Wasser und mit gesättigter Kochsalzlösung gewaschen und über Magnesiumsulfat getrocknet. Man erhält 107,15 g Rohprodukt. Dieses Rohprodukt wird destilliert. (Kp 117-140°/0,133 Pa (0.001 mm Hg) Heizbad 136-185°). Man erhält auf diese Weise, 86 g 4-(2-Acetoxybutyliden)-3,5,5-trimethyl-2-cyclohexen-1-on [Z/E = 73/26] entsprechend 61,4% d.Th. bezogen auf (2,6,6-Trimethyl-4-oxo-2-cyclohexen-1-yliden) acetaldehyd.

e) Die hier benützte Pyrolyseapparatur besteht aus:

α) Vorheizrohr: Pyrex, 30 cm, 2,5 cm ϕ, Raschigringe 7x7 mm, Heizmantel.

β) Hauptheizrohr: Quarz, 32 cm, 2,5 cm ϕ, Raschigringe 6x6 mm, Muffelofen.

90 g (0,36 M) 4-(2-Acetoxybutyliden)-3,5,5-trimethyl-2-cyclohexen-1-on werden unter einem Stickstoffstrom von 6 ml/Minute und am Wassserstrahlvakuum 1463 Pa (11 mm Hg) bei einer Zutropfgeschwindigkeit von 3-5 Tropfen pro Minute innerhalb von 6 Stunden pyrolysiert (Vorheizrohr 190°, Hauptheizrohr 500°). Das Reaktionsprodukt wird in einem auf -70°C abgekühlten Kolben aufgefangen. Zwei Kopf-Fraktionen werden anschliessend über eine 10 cm Widmerkolonne destilliert und charakterisieren sich wie folgt:

Fraktion 1: Bad 128°; 0,133 Pa (0,001 mmHg); Sd 40-98°; 4,9 g

Fraktion 2: Bad 128°; 0,133 Pa (0,001 mmHg); Sd 98°; 5,1 g

Der Rückstand wird bei 120° /13.33 Pa (0,1 mmHg) kurz wegdestilliert. Man erhält auf diese Weise 55,1 g (80%) 4-(2-Butenyliden)-3,5,5-trimethyl-2-cyclohexen-1-on.

Isomerenverhältnis: 19,48% ZZ, 41,30% ZE, 12,34% EZ, 26,86% EE.

## Patentansprüche

### Patentansprüche für folgende Vertragsstaaten : CH, DE, FR, GB, IT, LI, NL

1. Verbindungen der Formel

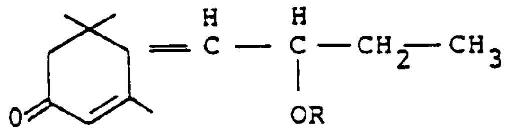

II

worin R Wasserstoff, $C_{1-6}$-Alkanoyl, gegebenenfalls substituiertes Benzoyl, $C_{1-6}$-Alkyl-oxycarbonyl oder Aryl-oxycarbonyl darstellt.

2. Verbindungen der Formel II gemäss Anspruch 1, dadurch gekennzeichnet, dass R Wasserstoff, $C_{1-6}$-Alkanoyl oder gegebenenfalls substituiertes Benzoyl darstellt.

3. Gemische der E- und der Z-Isomeren einer Verbindung II.

4. Gemische der E- und der Z-Isomeren einer Verbindung II im Verhältnis von 25-35 : 65-75.

5. Verfahren zur Herstellung der 4 geometrischen Isomeren von 4-(2-Butenyliden)-3,5,5-trimethyl-2-cyclohexen-1-on, dadurch gekennzeichnet, dass man eine Verbindung der Formel II gemäss Anspruch 1, worin R $C_{1-6}$-Alkyl-oxycarbonyl, Aryl-oxycarbonyl, oder, vorzugsweise $C_{1-6}$-Alkanoyl oder gegebenenfalls substituiertes Benzoyl darstellt, pyrolysiert.

6. Verfahren nach Anspruch 5, dadurch gekennzeichnet, dass man ein Gemisch des E- und Z-Isomeren der Verbindung II, insbesondere im Verhältnis von 25-35 : 65-75% pyrolysiert.

7. Verfahren nach Anspruch 5 oder 6, dadurch gekennzeichnet, dass man bei Temperaturen von 400°C bis 600°C pyrolysiert.

8. Verfahren nach Anspruch 6 oder 7, dadurch gekennzeichnet, dass man die eingesetzte Verbindung der Formel II durch Veresterung von 4-(2-Hydroxybutyliden) -3,5,5-trimethyl-2-cyclohexen-1-on gewinnt.

9. Verfahren nach Anspruch 8, dadurch gekennzeichnet, dass man als Hydroxyverbindung ein Gemisch der E- und Z-Isomeren, insbesondere im Verhältnis des Anspruchs 6 verestert.

10. Verfahren nach Anspruch 8 und 9, dadurch gekennzeichnet, dass man 4-(2-Hydroxybutyliden)-3,5,5-trimethyl-2-cyclohexen-1-on durch Umsetzen von (2,6,6-Trimethyl-4-oxo-2-cyclohexen-1-yliden)acetaldehyd mit einem organometallischen Reagens gewinnt.

11. Verfahren nach Anspruch 10, dadurch gekennzeichnet, dass man als organometallisches Reagens ein Reagens, das
    durch Umsetzung von $C_2H_5MgX$ mit einem Reagens der Formel $MeX_2$,
    durch Umsetzung von $C_2H_5MgX$ mit $(C_2H_5)_2$ Me oder
    durch Umsetzung mit $(C_2H_5)_2$ Me
        worin Me = Zn oder Cadmium und X = Chlor, Brom oder Jod sind,
    herstellbar ist,
    verwendet.

12. Verfahren nach Anspruch 8, dadurch gekennzeichnet, dass man ein Gemisch der E- und Z-Isomeren von (2,6,6-Trimethyl-4-oxo-2-cychlohexen-1-yliden)acetaldehyd, insbesondere im Verhältnis von 25-35 : 65-75 % als Ausgangsmaterial verwendet.

**Patentansprüche für folgenden Vertragsstaat : ES**

1. Verfahren zur Herstellung der 4 geometrischen Isomeren von 4-(2-Butenyliden)-3,5,5-trimethyl-2-cyclohexen-1-on, dadurch gekennzeichnet, dass man eine Verbindung der Formel

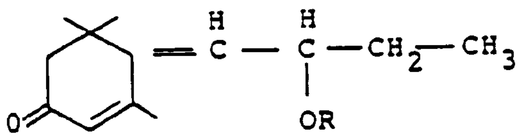

II

worin R $C_{1-6}$-Alkanoyl, gegebenenfalls substituiertes Benzoyl, $C_{1-6}$-Alkyl-oxycarbonyl oder Aryl-oxycarbonyl darstellt,
pyrolysiert.

2. Verfahren nach Anspruch 1, dadurch gekennzeichnet, dass man ein Gemisch des E- und Z-Isomeren der Verbindung II, insbesondere im Verhältnis von 25-35 : 65-75% pyrolysiert.

3. Verfahren nach Anspruch 1 oder 2, dadurch gekennzeichnet, dass man bei Temperaturen von 400°C bis 600°C pyrolysiert.

4. Verfahren nach Anspruch 2 oder 3, dadurch gekennzeichnet, dass man die eingesetzte Verbindung der Formel II durch Veresterung von 4-(2-Hydroxybutyliden)-3,5,5-trimethyl-2-cyclohexen-1-on gewinnt.

5. Verfahren nach Anspruch 4, dadurch gekennzeichnet, dass man als Hydroxyverbindung ein Gemisch der E- und Z-Isomeren, insbesondere im Verhältnis des Anspruchs 2 verestert.

6. Verfahren nach Anspruch 4 und 5, dadurch gekennzeichnet, dass man 4-(2-Hydroxybutyliden)-3,5,5-trimethyl -2-cyclohexen-1-on durch Umsetzen von (2,6,6-Trimethyl-4-oxo-2-cyclohexen-1-yliden)acetaldehyd mit einem organometallischen Reagens gewinnt.

7. Verfahren nach Anspruch 6, dadurch gekennzeichnet, dass man als organometallisches Reagens ein Reagens, das
   durch Umsetzung von $C_2H_5MgX$ mit einem Reagens der Formel $MeX_2$,
   durch Umsetzung von $C_2H_5MgX$ mit $(C_2H_5)_2$ Me oder
   durch Umsetzung mit $(C_2H_5)_2$ Me
      worin Me = Zn oder Cadmium und X = Chlor, Brom oder Jod sind,
   herstellbar ist,
   verwendet.

8. Verfahren nach Anspruch 7, dadurch gekennzeichnet, dass man ein Gemisch der E- und Z-Isomeren von (2,6,6-Trimethyl-4-oxo-2-cyclohexen-1-yliden)acetaldehyd, insbesondere im Verhältnis von 25-35 : 65-75 % als Ausgangsmaterial verwendet.

9. Verfahren nach einem der Ansprüche 1 bis 8, dadurch gekennzeichnet, dass R $C_{1-6}$ Alkanoyl oder gegebenenfalls substituiertes Benzoyl darstellt.

## Claims

### Claims for the following Contracting States : CH, DE, FR, GB, IT, LI, NL

1. Compounds of the formula

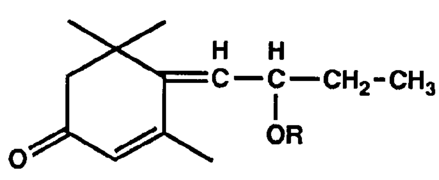

II

wherein R represents hydrogen, $C_{1-6}$-alkanoyl, optionally substituted benzoyl, $C_{1-6}$-alkyl-oxycarbonyl or

aryloxycarbonyl.

2. Compounds according to claim 1, wherein R represents hydrogen, $C_{1-6}$-alkanoyl or optionally substituted benzoyl.

3. A mixture of the E- and the Z-isomers of a compound II.

4. A mixture of the E- and the Z-isomers of a compound II in the ratio of 25-35:65-75.

5. A process for the manufacture of the 4 geometric isomers of 4-(2-butenylidene)-3,5,5-trimethyl-2-cyclohexen-1-one, characterized by pyrolyzing a compound of formula II in accordance with claim 1 in which R represents $C_{1-6}$-alkyloxycarbonyl, aryl-oxycarbonyl or, preferably, $C_{1-6}$-alkanoyl or optionally substituted benzoyl.

6. A process according to claim 5, characterized in that a mixture of the E- and Z-isomers of the compound II, espiecally in the ratio of 25-35 to 35-65%, is pyrolyzed.

7. A process according to claim 5 or 6, characterized in that the pyrolysis is carried out at temperatures of 400°C to 600°C.

8. A process according to claims 6 or 7, characterized in that the compound of formula II which is used is obtained by esterifying 4-(2-hydroxybutylidene)-3,5,5-trimethyl-2-cyclohexen-1-one.

9. A process according to claim 8, characterized in that a mixture of the E- and Z-isomers, especially in the ratio of claim 6, is esterified as the hydroxy compound.

10. A process according to claim 8 and 9, characterized in that 4-(2-hydroxybutylidene)-3,5,5-trimethyl-2-cyclohexen-1-one is obtained by reacting (2,6,6-trimethyl-4-oxo-2-cyclohexen-1-ylidene)acetaldehyde with an organometailic reagent.

11. A process according to claim 10, characterized in that there is used as the organometallic reagent a reagent which can be prepared
   by the reaction of $C_2H_5MgX$ with a reagent of the formula $MeX_2$,
   by the reaction of $C_2H_5MgX$ with $(C_2H_5)_2Me$ or
   by reaction with $(C_2H_5)_2M$ e
      wherein Me is zinc or cadmium and X is chlorine, bromine or iodine.

12. A process according to claim 8, characterized in that a mixture of the E- and Z-isomers of (2,6,6-trimethyl-4-oxo-2-cyclohexen-1-ylidene)acetaldehyde, especially in the ratio of 25-35:35-65%, is used as the starting material.

**Claims for the following Contracting State : ES**

1. A process for the manufacture of the 4 geometric isomers of 4-(2-butenylidene)-3,5,5-trimethyl-2-cyclohexene-1-one, characterized by pyrolyzing a compound of the formula

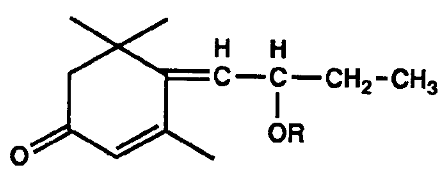

II

wherein R represents $C_{1-6}$-alkanoyl, optionally substituted benzoyl, $C_{1-6}$-alkyl-oxycarbonyl or aryl-oxycarbonyl.

2. A process according to claim 1, characterized in that a mixture of the E- and Z-isomers of the compound II, especially in the ratio of 25-35:35-65%, is pyrolyzed.

3. A process according to claims 1 or 2, characterized in that the pyrolysis is carried out at temperatures of

400°C to 600°C.

4. A process according to claim 2 or 3, characterized in that the compound of formula II which is used is obtained by esterifying 4-(2-hydroxybutylidene)-3,5,5-trimethyl-2-cyclohexen-1-one.

5. A process according to claim 4, characterized in that a mixture of the E- and Z-isomers, especially in the ratio of claim 2, is esterified as the hydroxy compound.

6. A process according to claim 4 and 5, characterized in that 4-(2-hydroxybutylidene)-3,5,5-trimethyl-2-cyclohexen-1-one is obtained by reacting (2,6,6-trimethyl-4-oxo-2-cyclohexen-1-ylidene)acetaldehyde with an organometallic reagent.

7. A process according to claim 6, characterized in that there is used as the organometailic reagent a reagent which can be prepared

   by the reaction of $C_2H_5MgX$ with a reagent of the formula $MeX_2$,

   by the reaction of $C_2H_5MgX$ with $(C_2H_5)_2Me$ or

   by reaction with $(C_2H_5)_2M$ e

      wherein Me is zinc or cadmium and X is chlorine, bromine or iodine.

8. A process according to claim 7, characterized in that a mixture of the E- and Z-isomers of (2,6,6-trimethyl-4-oxo-2-cyclohexen-1-ylidene)acetaldehyde, especially in the ratio of 25-35:35-65%, is used as the starting material.

9. A process according to any one of claims 1 to 8, characterized in that R represents $C_{1-6}$ alkanoyl or optionally substituted benzoyl.

**Revendications**

**Revendications pour les Etats contractants suivants : CH, DE, FR, GB, IT, NL, LI**

1. Composés de formule

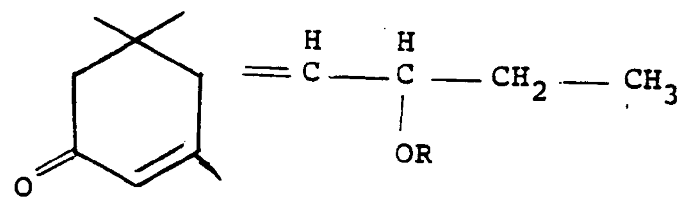

II

dans laquelle R représente l'hydrogène, un groupe alcanoyle en $C_1$-$C_6$, un groupe benzoyle éventuellement substitué, un groupe alkyle en $C_1$-$C_6$-oxycarbonyle ou aryloxycarbonyle.

2. Composés de formule II de la revendication 1, caractérisés en ce que R représente l'hydrogène,un groupe alcanoyle en $C_1$-$C_6$ ou un groupe benzoyle éventuellement substitué.

3. Mélanges des isomères E et Z d'un composé II.

4. Mélanges des isomères E et Z d'un composé II dans des proportions relatives de 25 à 35 : 65 à 75.

5. Procédé de préparation des quatre isomères géométriques de la 4-(2-buténylidène)-3,5,5-triméthyl-2-cyclohexène-1-one, caractérisé en ce que l'on pyrolyse un composé de formule II de la revendication 1 dans laquelle R représente un groupe alkyle en $C_1$-$C_6$-oxycarbonyle, aryl-oxycarbonyle, ou bien, de préférence, un groupe alcanoyle en C1-C6 ou benzoyle éventuellement substitué.

6. Procédé selon la revendication 5, caractérisé en ce que l'on pyrolyse un mélange des isomères E et Z du composé II, en particulier dans des proportions relatives de 25 à 35 : 65 à 75 %.

7. Procédé selon la revendication 5 ou 6, caractérisé en ce que l'on pyrolyse à des températures de 400 à 600°C.

8. Procédé selon la revendication 6 ou 7, caractérisé en ce que le composé de formule II mis en oeuvre est obtenu par estérification de la 4-(2-hydroxybutylidène)-3,5,5-triméthyl-2-cyclohexène-1-one.

9. Procédé selon la revendication 8, caractérisé en ce que le dérivé hydroxylé soumis à estérification est un mélange des isomères E et Z, en particulier dans les proportions relatives de la revendication 6.

10. Procédé selon les revendications 8 et 9, caractérisé en ce que l'on obtient la 4-(2-hydroxybutylidène)-3,5,5-triméthyl-2-cyclohexène-1-one par réaction du (2,6,6-triméthyl-4-oxo-2-cyclohexène-1-ylidène)acétaldéhyde avec un réactif organométallique.

11. Procédé selon la revendication 10, caractérisé en ce que l'on utilise en tant que réactif organométallique un réactif pouvant être préparé
par réaction de $C_2H_5MgX$ avec un réactif de formule $MeX_2$,
par réaction de $C_2H_5MgX$ avec $(C_2H_5)_2Me$ ou
par réaction avec $(C_2H_5)_2Me$,
Me représentant le zinc ou le cadmium et X le chlore, le brome ou l'iode.

12. Procédé selon la revendication 8, caractérisé en ce que l'on utilise en tant que produit de départ un mélange des isomères E et Z du (2,6,6-triméthyl-4-oxo-2-cyclohexène-1-ylidène)-acétaldéhyde, en particulier dans des proportions relatives de 25 à 35 : 65 à 75 %.

**Revendications pour l'Etat Contractant suivant : ES**

1. Procédé de préparation des quatre isomères géométriques de la 4-(2-buténylidène)-3-5,5-triméthyl-2-cyclohexène-1-one, caractérisé en ce que l'on pyrolyse un composé de formule

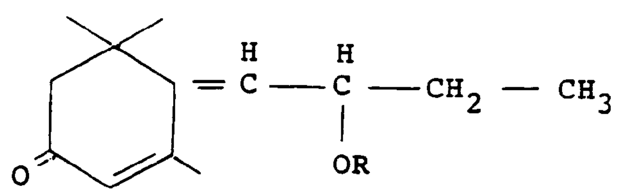

II

dans laquelle R représente un groupe alcanoyle en $C_1$-$C_6$, un groupe benzoyle éventuellement substitué, un groupe alkyle en $C_1$-$C_6$ -oxycarbonyle ou aryl-oxycarbonyle.

2. Procédé selon la revendication 1, caractérisé en ce que l'on pyrolyse un mélange des isomères E et Z du composé II, en particulier dans des proportions relatives de 25 à 35 : 65 à 75 %.

3. Procédé selon la revendication 1 ou 2, caractérisé en ce que l'on pyrolyse à des températures de 400 à 600°C.

4. Procédé selon la revendication 2 ou 3, caractérisé en ce que le composé de formule II mis en oeuvre est obtenu par estérification de la 4-(2-hydroxybutylidène)-3,5,5-triméthyl-2-cyclohexène-1-one.

5. Procédé selon la revendication 4, caractérisé en ce que le dérivé hydroxylé soumis à estérification est un mélange des isomères E et Z, en particulier dans les proportions relatives de la revendication 2.

6. Procédé selon les revendications 4 et 5, caractérisé en ce que l'on obtient la 4-(2-hydroxybutylidène)-3,5,5-triméthyl-2-cyclohexène-1-one par réaction du (2,6,6-triméthyl-4-oxo-2-cyclohexène-1-ylidène)-acétaldéhyde avec un réactif organométallique.

7. Procédé selon la revendication 6, caractérisé en ce que l'on utilise en tant que réactif organométallique un réactif pouvant être préparé
par réaction de $C_2H_5MgX$ avec un réactif de formule $MeX_2$,

par réaction de $C_2H_5MgX$ avec $(C_2H_5)_2Me$ ou
par réaction avec $(C_2H_5)_2Me$,
Me représentant le zinc ou le cadmium et X le chlore, le brome ou l'iode.

**8.** Procédé selon la revendication 7, caractérisé en ce que l'on utilise en tant que produit de départ un mélange des isomères E et Z du (2,6,6-triméthyl-4-oxo-2-cyclohexène-1-ylidène)-acétaldéhyde, en particulier dans des proportions relatives de 25 à 35 : 65 à 75 %.

**9.** Procédé selon l'une des revendications 1 à 8, caractérisé en ce que R représente un groupe alcanoyle en $C_1$-$C_6$ ou un groupe benzoyle éventuellement substitué.